# EUROPÄISCHE PATENTSCHRIFT

(11) **EP 4 182 255 B1**
(45) Veröffentlichungstag und Bekanntmachung des Hinweises auf die Patenterteilung: **01.05.2024**
(21) Anmeldenummer: 21734110.6
(22) Anmeldetag: 17.06.2021
(51) Int. Cl.: B66B 31/02

(54) **HANDLAUF-BEHANDLUNGSVORRICHTUNG FÜR PERSONENTRANSPORTANLAGEN**
HANDRAIL TREATMENT DEVICE FOR PERSONAL TRANSPORT SYSTEMS
DISPOSITIF DE MANIPULATION DE MAIN COURANTE POUR INSTALLATIONS DE TRANSPORT DES PERSONNES

(30) Priorität: 17.07.2020 EP 20186516
(43) Veröffentlichungstag der Anmeldung: 24.05.2023
(73) Patentinhaber: INVENTIO AG, 6052 Hergiswil (CH)
(72) Erfinder: BAUER, Wolfgang, 7222 Rohrbach (AT); HAIDER, Michael, 7033 Pöttsching (AT); NESZMERAK, Wolfgang, 1120 Wien (AT)
(74) Vertreter: Inventio AG
(86) Internationale Anmeldenummer: PCT/EP2021/066518
(87) Internationale Veröffentlichungsnummer: WO 2022/012856

(56) Entgegenhaltungen:
- JP-A- H08 188 369
- KR-A- 20110 023 060

## Beschreibung

Die vorliegende Erfindung betrifft eine Handlauf-Behandlungsvorrichtung, umfassend zumindest ein Gehäuse, ein rotierender Reinigungsbesatz und eine Strahlungsquelle, welche keimtötende elektromagnetische Strahlung emittieren kann, sowie eine Personentransportanlage mit mindestens einer Handlauf-Behandlungsvorrichtung und ein Verfahren zum Steuern der Handlauf-Behandlungsvorrichtung.

Personentransportanlagen die als Fahrtreppen oder Fahrsteige ausgestalten sind, weisen in der Regel umlaufende Handläufe auf, die sich mit gleicher Geschwindigkeit bewegen wie ein Transportband, auf dem die Benutzer stehen können. Dadurch können sich die Benutzer während der Fahrt an den Handläufen festhalten. Der Bereich des Handlaufs, bei dem sich die Benutzer festhalten können, wird als vorlaufendes Trum bezeichnet, während dessen rückführendes Trum in der Regel vor den Benutzern der Personentransportanlage verdeckt, entgegen der Transportrichtung zurückgeführt wird.

Durch die Berührung der Hände der Benutzer mit der Oberfläche der Handläufe gelangen Bakterien, Sporen und Viren an den Handlauf. Die so abgelagerten Krankheitserreger können sich auf dem Handlauf vermehren und auf nachfolgende Benutzer übertragen werden. Der Handlauf einer solchen Personentransportanlage stellt somit einen unhygienischen Ort im öffentlichen Raum dar, an dem ansteckende Krankheiten übertragen werden können.

Die WO2019/164256A1 zeigt eine Handlauf-Behandlungsvorrichtung gemäß dem Stand der Technik. Bei dieser wird ein Handlauf mittels Strahlungsquellen mit ultraviolettem Licht bestrahlt. Diese keimtötende elektromagnetische Strahlung zerstört die Desoxyribonukleinsäure dieser Keime so dass auf der Oberfläche des Handlaufs ab der Austrittsseite dieser Handlauf-Behandlungsvorrichtung eine signifikante Reduktion der Mikroorganismen feststellbar ist. Allerdings kann am Handlauf klebender Schmutz den Bakterien und Viren ausreichend Schutz bieten, so dass sie die Strahlungsbehandlung unbeschadet überstehen können.

Um eine noch gründlichere Desinfizierung zu ermöglichen, sieht beispielsweise die JP08188369A eine umfangreiche Vorreinigung mit rotierenden Bürsten und einer Waschanlage mit keimtötenden Fluiden vor, so dass die gereinigte Oberfläche des Handlaufs hernach durch die Strahlung der UV-Lampe desinfiziert werden kann. Allerdings ist eine solche Handlauf-Behandlungsvorrichtung sehr teuer und verursacht insbesondere wegen den erforderlichen Fluiden einen grossen Betriebsaufwand.

Aufgabe der vorliegenden Erfindung ist es daher, mittels einer einfachen Handlauf-Behandlungsvorrichtung die an den Handläufen anhaftenden Krankheitserreger kostengünstig und sicher, unschädlich zu machen.

Diese Aufgabe wird gelöst durch eine Handlauf-Behandlungsvorrichtung, welche zumindest ein Gehäuse und ein im Gehäuse um eine Drehachse drehbar angeordneter, zylindrischer Grundkörper umfasst. An der Zylinderaussenfläche des zylindrischen Grundkörpers ist ein Reinigungsbesatz angeordnet, über welchen Reinigungsbesatz zwecks Entfernung von Schmutzpartikeln ein umlaufender Handlauf einer Fahrtreppe oder eines Fahrsteiges führbar ist. Des Weiteren ist innerhalb des Gehäuses mindestens eine Strahlungsquelle angeordnet, die keimtötende elektromagnetische Strahlung emittieren kann. Die Strahlungsquelle ist derart auf den Grundkörper ausgerichtet, dass bei drehendem Grundkörper, der sich kontinuierlich an der Strahlungsquelle vorbeibewegende Reinigungsbesatz der keimtötenden elektromagnetischen Strahlung dieser Strahlungsquelle ausgesetzt ist.

Die zu verwendende keimtötende elektromagnetische Strahlung hat vorzugsweise einen Wellenlängenbereich von 200nm bis 280nm, besonders bevorzugt von 250nm bis 260nm, und kann beispielsweise mittels UV-C Röhren oder UV-C Leuchtdioden erzeugt werden. Der Reinigungsbesatz kann ein Borstenbesatz, ein aus elastischem Material bestehender Lamellenbesatz und dergleichen mehr sein.

Dadurch dass der Reinigungsbesatz kontinuierlich der elektromagnetischen keimtötenden Strahlung ausgesetzt ist, werden am Reinigungsbesatz anhaftende Bakterien und Viren abgetötet, bevor dessen kontaminierte Bereiche wieder in Kontakt mit dem Handlauf gelangen. Mit anderen Worten ausgedrückt, wird durch das Bestrahlen des Reinigungsbesatzes vermieden, dass diese Keime durch einen nicht entkeimten Reinigungsbesatz beispielsweise in feine Risse und Vertiefungen des Handlaufs eingearbeitet werden und sich dann trotzdem auf die Benutzer der Fahrtreppe oder des Fahrsteiges übertragen können. Durch das mechanische Reinigen des Handlaufs mit einem desinfizierten, rotierenden Reinigungsbesatz gemäss dem vorliegenden Erfindungsgegenstand, werden die Oberflächen des Handlaufs von daran anhaftendem, mit Keimen durchsetztem Schmutz gereinigt, so dass insgesamt der Keimdruck (Anzahl vorhandene Keime auf diesen Oberflächen) verringert wird.

In Bezug auf die Form des Grundkörpers bedeutet zylindrisch lediglich, dass es sich hierbei um einen länglichen, drehbar gelagerten Körper handelt. Dieser ist vorzugsweise rotationssymmetrisch ausgebildet, so dass möglichst wenig Unwuchten und Vibrationen am darüber geführten Handlauf verursacht werden. Der Grundkörper muss auch nicht zwingend formstarr wie beispielsweise eine Walze aus Stahl sein, sondern kann auch aus einem elastischeren Material wie faserverstärkter Kunststoff oder sogar aus einem weichelastischen Material wie beispielsweise Elastomeren gefertigt sein, sofern sich dieses gut drehbar im Gehäuse lagern lässt. Logischerweise ist auch ein Verbund verschiedener Materialien möglich, um den Grundkörper aufzubauen und diesen mit vorteilhaften Eigenschaften wie Formstarre in den Lagerstellen und höheren elastischen Eigenschaften im Bereich des Reinigungsbesatzes zu versehen.

Im Gehäuse der Handlauf-Behandlungsvorrichtung kann mindestens ein Abstreifelement vorhanden sein, dessen Abstreifkante mit Reinigungsspitzen des Reinigungsbesatzes zusammenwirkt. Diese Reinigungsspitzen sind bei einem Borstenbesatz die Borstenspitzen, bei einem Lamellenbesatz die Lamellenspitzen und bei anderem Material, wie beispielsweise einem als Reinigungsbesatz dienender Schaumstoffring, dessen mit dem Handlauf in Kontakt stehende, poröse Oberfläche. Hierdurch wir der vom Handlauf entfernte und gegebenenfalls im Reinigungsbesatz feststeckende Schmutz kontinuierlich aus diesem entfernt, so dass hauptsächlich derart mechanisch gereinigte Bereiche des Reinigungsbesatzes wieder an den Handlauf gelangen.

In einer Ausführung kann das Abstreifelement eine Lippe beziehungsweise Lamelle aus biegeelastischem Material aufweisen. An dieser Lippe ist die Abstreifkante ausgebildet. Hierdurch werden die mit der Lippe in Kontakt geratenden Reinigungsspitzen geringeren mechanischen Kräften ausgesetzt und dadurch deren Lebensdauer erhöht. Zudem erzeugen die an der Lippe vorbeistreichenden Reinigungsspitzen Vibrationen, die das Abschütteln des Schmutzes aus dem Reinigungsbesatz zwar begünstigen, sich jedoch durch die biegeelastisch ausgebildete Lippe nur gedämpft auf das Gehäuse übertragen. Dadurch lässt sich die Geräuschentwicklung der Handlauf-Behandlungsvorrichtung im Betrieb erheblich reduzieren.

In einer alternativen Ausführung kann das Abstreifelement eine Abstreifbürste aufweisen, deren Borstenspitzen die Abstreifkante bilden. Die wirkbezogenen Eigenschaften der Abstreifbürste sind im Wesentlichen dieselben wie bei der vorangehend beschriebenen Ausführung des Abstreifelementes mit einer Lippe. Je nach Ausgestaltung des Reinigungsbesatzes (Borstenstärke, Borstendichte, Anordnung der Borsten beziehungsweise von Borstenbündeln, der Form und Steifigkeit der Lamellen, etc.) reinigt die Ausführung mit einer Lippe oder die Ausführung mit einer Abstreifbürste den Reinigungsbesatz besser, so dass die Auswahl hier vorzugsweise experimentell getroffen wird.

In einer Ausführung der Handlauf-Behandlungsvorrichtung kann der zylindrische Grundkörper durch die Bewegung des über diesen geführten Handlauf angetrieben werden. Um eine ausreichende Reinigungswirkung zu erzielen, ist jedoch eine Geschwindigkeitsdifferenz zwischen der Oberfläche des Handlaufs und den Reinigungsspitzen des Reinigungsbesatzes erforderlich. Eine solche Geschwindigkeitsdifferenz kann dadurch erreicht werden, dass die Rotationsbewegung des Grundkörpers beziehungsweise des Reinigungsbesatzes kontinuierlich gebremst wird, ohne aber diesen vollständig zu blockieren. Hierzu eignet sich insbesondere das Abstreifelement, welches derart mit den Reinigungsspitzen des Reinigungsbesatzes zusammenwirkt, dass bei bewegtem Handlauf dadurch eine Bremswirkung am zylindrischen Grundkörper erfolgt und infolgedessen zwischen der Oberfläche des Handlaufs und den diesen berührenden Reinigungsspitzen eine Differenzgeschwindigkeit vorhanden ist.

Alternativ dazu kann der zylindrische Grundkörper auch durch einen Motor angetrieben werden. Hierdurch lässt sich die die Drehgeschwindigkeit des zylindrischen Grundkörpers derart auf die Geschwindigkeit des Handlaufs abstimmen, dass bei bewegtem Handlauf zwischen der Oberfläche des Handlaufs und den diesen berührenden Reinigungsspitzen eine Differenzgeschwindigkeit vorhanden ist. Diese Alternative hat den Vorteil, dass die Drehgeschwindigkeit des Grundkörpers auch wesentlich höher als die Geschwindigkeit des darüber geführten Handlaufs sein kann, so dass zur Entfernung von Schmutz aus dem Reinigungsbesatz eine auf die Schmutzpartikel wirkende, höhere Zentrifugalkraft ausgenutzt werden kann. Auch entgegengesetzte Bewegungsrichtungen sind bei dieser alternativen Ausgestaltung möglich.

In einer weiteren Ausgestaltung der Erfindung kann der Reinigungsbesatz orthogonal zur Drehachse profiliert sein, so dass eine Laufflächenkontur des Reinigungsbesatzes mit einer Aussenflächenkontur des darüber zu führenden beziehungsweise zu reinigenden Handlaufes korrespondiert. Hierdurch wird nicht nur die breite Hauptfläche, auf der die Handballen der Benutzer üblicherweise aufliegen, gereinigt, sondern auch die beiden seitlichen Flächen, die üblicherweise von den Fingern der Benutzer umgriffen werden, durch den Reinigungsbesatz erreicht und abgebürstet.

In einer weiteren Ausgestaltung der Erfindung kann der zylindrische Grundkörper becherförmig ausgebildet sein und dessen Zylindermantel Durchbrüche aufweisen. Der Reinigungsbesatz ist hierbei an der vorhandenen beziehungsweise verbliebenen Zylinderaussenfläche des Zylindermantels angeordnet. Das Innere des becherförmig ausgebildeten Grundkörpers ist zudem durch die Strahlungsquelle oder eine weitere Strahlungsquelle derart ausleuchtbar, dass deren keimtötende elektromagnetische Strahlung durch die Durchbrüche hindurch zum Reinigungsbesatz gelangt. Durch diese Weiterbildung der Erfindung kann der Reinigungsbesatz auch in der Tiefe durch die keimtötende elektromagnetische Strahlung erreicht und desinfiziert werden.

In einer weiteren Ausgestaltung der Handlauf-Behandlungsvorrichtung kann ein Schmutzschutz für die mindestens eine Strahlungsquelle im Gehäuse angeordnet sein, damit sich im Gehäuse umherwirbelnder Schmutz nicht direkt auf der Strahlungsquelle festsetzt. Ein solcher Schmutzschutz können beispielsweise eine transparente Abdeckung der Strahlungsquelle oder Leitkörper jeglicher Art sein, die die Schmutzpartikel von der Strahlungsquelle fernhalten. Der Schmutzschutz kann aber beispielsweise auch eine Absaugvorrichtung ähnlich einem Staubsauger sein, welche die herumwirbelnden Schmutzpartikel direkt absaugt und somit aus dem Bereich der Strahlungsquelle und dem zylindrischen Grundkörper entfernt, bevor sich diese im Gehäuse niedersetzen und ansammeln können.

In einer weiteren Ausgestaltung der Handlauf-Behandlungsvorrichtung kann auch eine Reinigungsvorrichtung für die mindestens eine Strahlungsquelle im Gehäuse angeordnet sein. Hierzu gibt es verschiedene Möglichkeiten wie beispielsweise auf die Strahlungsquelle gerichtete Druckluftdüsen, elektromechanisch oder händisch zu betätigende Wischersysteme und dergleichen mehr.

In einer weiteren Ausgestaltung der Handlauf-Behandlungsvorrichtung kann in Bezug auf die Bewegungsrichtung des Handlaufs eine dem zylindrischen Grundkörper mit Reinigungsbesatz nachgeschalteter Bereich mit Strahlungsquellen vorhanden sein. Mittels deren keimtötenden elektromagnetischen Strahlung kann zumindest ein Teil der Handlaufoberfläche des Handlaufs bestrahlt werden. Ein derart behandelter Handlauf verlässt die Handlauf-Behandlungsvorrichtung nahezu keimfrei. Wenn für die Personentransportanlage beide möglichen Transportrichtungen beziehungsweise Bewegungsrichtungen vorgesehen sind, können auch zwei Bereiche mit Strahlungsquellen zum Bestrahlen der Handlaufoberfläche vorhanden sein, wobei der zylindrische Grundkörper dann zwischen den beiden Bereichen angeordnet ist. Um Energie zu sparen, können beispielsweise die beiden Bereiche bewegungsrichtungsabhängig angesteuert werden, so dass immer nur der dem zylindrischen Grundkörper nachfolgend angeordnete und daher nachgeschaltete Bereich keimtötende elektromagnetische Strahlung abgibt.

Da der drehende zylindrische Grundkörper mit seinem Reinigungsbesatz entfernte Schmutzpartikel herumwirbeln kann, weist die Handlauf-Behandlungsvorrichtung vorzugsweise ein im Wesentlichen geschlossenes Gehäuse auf, durch welches der Handlauf über eine Eintrittsöffnung und eine Austrittsöffnung durchgeführt wird. Um den Handlauf bei einem Wechsel einfacher durchführen zu können, ist das Gehäuse vorzugsweise zweiteilig ausgeführt und weist ein U-förmiges Hauptgehäuseteil und eine, die offene Seite des Hauptgehäuseteils überspannende Abdeckung auf.

Eine Personentransportanlage die als Fahrtreppe oder Fahrsteig ausgebildet ist, weist mindestens eine Balustrade auf, die mindestens einen umlaufenden Handlauf umfasst, an dem sich die Benutzer während der Fahrt festhalten können. Üblicherweise sind zwei Balustraden vorhanden, die sich zu beiden Seiten eines Transportbandes der Personentransportanlage erstrecken. Vorzugsweise ist für jeden umlaufenden Handlauf mindestens eine erfindungsgemässe Handlauf-Behandlungsvorrichtung vorgesehen.

In den meisten Fällen umfasst die Balustrade einen Balustradensockel, in dem ein rückführendes Trum des Handlaufs vor den Benutzern der Personentransportanlage verdeckt, geführt ist. Der Balustradensockel bietet daher die Möglichkeit, die Handlauf-Behandlungsvorrichtung ebenfalls von den Blicken der Benutzer verdeckt, im Innern des Balustradensockels der Balustrade zu installieren.

Bekanntermassen weist eine als Fahrsteig oder Fahrtreppe ausgestaltete Personentransportanlage einen Antriebsmotor zum Antreiben des Handlaufs und eine Antriebssteuerung zum Steuern des Antriebsmotors auf. Die mindestens eine Strahlungsquelle der vorangehend beschriebenen Ausführungsvarianten der Handlauf-Behandlungsvorrichtung kann abhängig von einem Geschwindigkeitssignal und/oder Bewegungsrichtungssignal der Antriebssteuerung an den Antriebsmotor, angesteuert werden. Hierdurch kann beispielsweise erreicht werden, dass die mindestens eine Strahlungsquelle nur dann keimtötende elektromagnetische Strahlung aussendet, wenn der Handlauf in Bewegung ist. Selbstverständlich kann auch die Drehgeschwindigkeit und/oder Drehrichtung des zylindrischen Grundkörpers in Abhängigkeit dieser Signale gesteuert werden, wenn dieser durch einen Motor angetrieben wird.

Nachfolgend werden Ausführungsformen der Erfindung unter Bezugnahme auf die beigefügten Zeichnungen beschrieben, wobei weder die Zeichnungen noch die Beschreibung als die Erfindung einschränkend, auszulegen sind. Ferner werden für gleiche oder gleichartig wirkende Elemente dieselben Bezugszeichen verwendet. Es zeigen:
- Figur 1:: eine vereinfachte Ansicht einer als Fahrtreppe ausgestalteten Personentransportanlage mit einer Handlauf-Behandlungsvorrichtung;
- Figur 2:: ein Querschnitt entlang der Linie A-A durch die Personentransportanlage der Figur 1;
- Figur 3:: in einer Frontansicht, die in den Figuren 1 und 2 dargestellte Handlauf-Behandlungsvorrichtung in einem ersten Ausführungsbeispiel;
- Figur 4:: ein Querschnitt entlang der Linie B-B durch die in der Figur 3 dargestellte Handlauf-Behandlungsvorrichtung;
- Figur 5:: in einer Frontansicht, die in den Figuren 1 und 2 dargestellte Handlauf-Behandlungsvorrichtung in einem zweiten Ausführungsbeispiel;
- Figur 6:: ein Querschnitt entlang der Linie C-C durch die in der Figur 5 dargestellte Handlauf-Behandlungsvorrichtung.

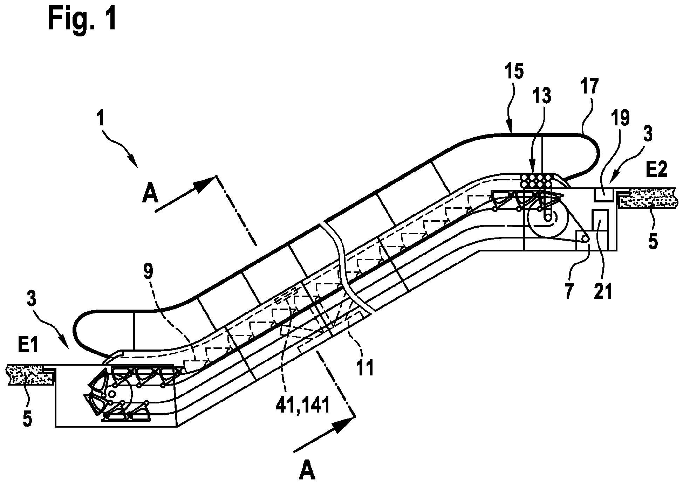

Figur 1 zeigt eine vereinfachte Ansicht einer Personentransportanlage 1 mit einem als Fachwerk ausgestalteten Tragwerk 11. Die als Fahrtreppe ausgestaltete Personentransportanlage 1 verbindet eine untere Ebene E1 mit einer oberen Ebene E2 eines Gebäudes 5. Die Personentransportanlage 1 kann von Benutzern über Zutrittsbereiche 3 betreten und wieder verlassen werden. Im Tragwerk 11 ist ein umlaufendes Stufenband 9 angeordnet, welches in der oberen Ebene E2 und in der unteren Ebene E1 umgelenkt wird und somit einen vorlaufenden Abschnitt und einen rücklaufenden Abschnitt aufweist. Der besseren Übersicht wegen wurde auf die detaillierte Darstellung des rücklaufenden Abschnitts verzichtet, ebenso auf eine detaillierte Darstellung von Spanten, Führungsschienen, und Schienenblöcken.

Die Personentransportanlage 1 weist ferner zwei Balustraden 15 auf, die sich jeder Längsseite des Stufenbandes 9 entlang erstrecken, wobei in Figur 1 nur die in der Betrachtungsebene im Vordergrund angeordnete Balustrade 15 sichtbar ist. An jeder Balustrade 15 ist ein Handlauf 17 umlaufend angeordnet, wobei dessen rücklaufendes Trum in einem Balustradensockel 13 geführt ist. Dieser Balustradensockel 13 verbindet die Balustrade 15 mit dem Tragwerk 11. Mit anderen Worten wird das rückführende Trum des Handlaufs 17 vor den Benutzern der Personentransportanlage 1 verdeckt, in dem Balustradensockel 13 geführt.

Das Stufenband 9 sowie der Handlauf 17 können durch eine Antriebseinheit 7 der Personentransportanlage 1 angetrieben werden, welche Antriebseinheit 7 einen Antriebsmotor 21 aufweist. Die Bewegungsrichtungen der Handläufe 17 und des Stufenbandes 9 sowie deren Geschwindigkeit werden durch eine Steuerung 19 vorgegeben, welche den Antriebsmotor 21 mittels Steuersignalen, beispielsweise über einen nicht dargestellten Frequenzumrichter, ansteuert.

Des Weiteren weist die Personentransportanlage 1 für jeden umlaufenden Handlauf 17 mindestens eine Handlauf-Behandlungsvorrichtung 41, 141 auf. Diese ist ebenfalls im Balustradensockel 13 der Balustrade 15 installiert und damit vor den Benutzern der Personentransportanlage 1 verdeckt.

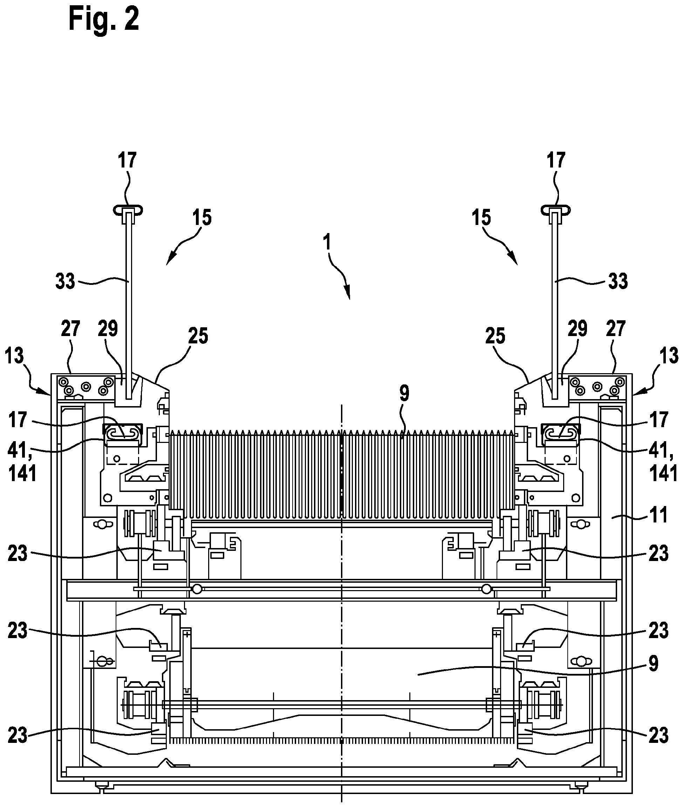

In Figur 2 ist ein Querschnitt durch die Personentransportanlage 1 gemäß Figur 1 entlang der Linie A-A dargestellt. In diesem Querschnitt ist sowohl der vorlaufende als auch der rücklaufende Abschnitt des Stufenbandes 9 erkennbar. Das Stufenband 9 wird innerhalb des Tragwerks 11 auf Führungsschienen 23 geführt. Links und rechts vom vorlaufenden, bei bestimmungsgemäßem Einbau der Personentransportanlage 1 oben angeordneten Abschnitt des Stufenbandes 9, ist je eine Balustrade 15 und ein Balustradensockel 13 angeordnet. Innerhalb des Balustradensockels 13 sind durch Verkleidungsbleche 25, 27 verdeckt, Klemmvorrichtungen 29 vorhanden, die als Klemmaufnahmen der einzelnen Balustradenpaneele 33 dienen. Hierbei ist das Balustradenpaneel 33 an seinem unteren Ende zumindest in einer der Klemmvorrichtungen 29 ortsfest eingespannt. Die Klemmvorrichtungen 29 sind entlang der Längserstreckung der Personentransportanlage 1 innerhalb des Balustradensockels 13 am Tragwerk 11 angeordnet. Unterhalb der Klemmvorrichtungen 29 ist für jeden an der Balustrade 15 umlaufend angeordneten Handlauf 17 eine Handlauf-Behandlungsvorrichtung 41, 141 vorgesehen.

Da im Balustradensockel 13 auch das rücklaufende Trum des Handlaufes 17 geführt ist, ist es von Vorteil, die Handlauf-Behandlungsvorrichtung 41, 141 im Balustradensockel 13 anzuordnen und dort das rücklaufende Trum je nach Ausgestaltung über oder durch die Handlauf-Behandlungsvorrichtung 41, 141 hindurch zu führen. Dadurch kann sichergestellt werden, dass Benutzer der Personentransportanlage 1 nicht mit der Handlauf-Behandlungsvorrichtung 41, 141 in Berührung gelangen können.

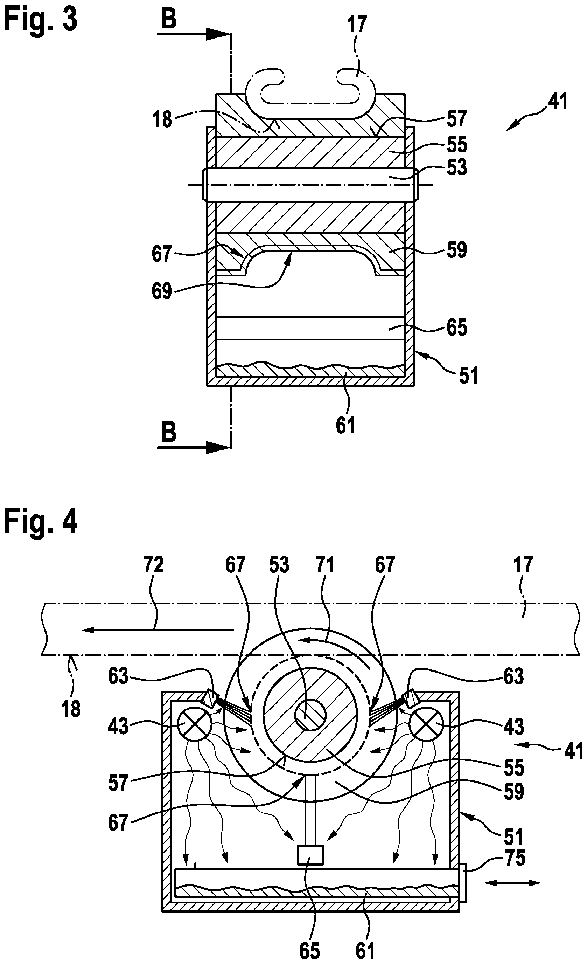

Die Figur 3 zeigt in einer Frontansicht ein erstes Ausführungsbeispiel der in den Figuren 1 und 2 dargestellten Handlauf-Behandlungsvorrichtung 41. Die Figur 4 zeigt einen Querschnitt entlang der Linie B-B durch die in der Figur 3 dargestellte Handlauf-Behandlungsvorrichtung 41. Nachfolgend werden die beiden Figuren 3 und 4 gemeinsam beschrieben.

Dieses Ausführungsbeispiel umfasst ein Gehäuse 51 und ein im Gehäuse 51 um eine Drehachse 53 drehbar angeordneter, zylindrischer Grundkörper 55. An einer Zylinderaussenfläche 57 des Grundkörpers 55 ist ein Reinigungsbesatz 59 angeordnet, über welchen Reinigungsbesatz 59 der Handlauf 17 geführt werden kann, damit Schmutzpartikel 61 von dessen Handlaufoberfläche 18 entfernt werden können. Durch die Bewegung des Handlaufs 17 wird infolge der Reibung zwischen dem Reinigungsbesatz 59 und der Handlaufoberfläche 18 der zylindrische Grundkörper 55 angetrieben.

Innerhalb des Gehäuses 51 sind zudem zwei Strahlungsquellen 43 angeordnet, die keimtötende elektromagnetische Strahlung emittieren können. Vorzugsweise werden hierzu Leuchtröhren oder Leuchtdioden verwendet, welche beispielsweise ultraviolettes Licht im Wellenlängenbereich von 200nm bis 280nm, sogenanntes UV-C Licht emittieren können, es sind aber auch andere Strahlungsquellen 43 möglich, die keimtötende Strahlung abgeben können. Logischerweise werden zur ausreichenden Bestrahlung jeweils eine Reihe von UV-C LED' s mit sich überschneidenden Leichtkegeln benötigt, um auf derselben Breite des Gehäuses 51 die Entkeimungswirkung wie eine der dargestellten UV-C Leuchtröhren zu erhalten.

Die beiden Strahlungsquellen 43 sind so auf den Grundkörper 55 ausgerichtet, dass bei drehendem Grundkörper 55 der sich kontinuierlich an den Strahlungsquellen 43 vorbeibewegende Reinigungsbesatz 59 der keimtötenden elektromagnetischen Strahlung dieser Strahlungsquellen 43 ausgesetzt ist. Hierdurch wird der Reinigungsbesatz 59 sowie der durch den Reinigungsbesatz 59 vom Handlauf abgetragene Schmutz entkeimt, so dass der Handlauf 17 kontinuierlich durch entkeimte Bereiche des Reinigungsbesatzes 59 gereinigt wird und nicht Bakterien und Viren durch den rotierenden Reinigungsbesatz 59 wieder auf die Handlaufoberfläche 18 aufgetragen werden.

Des Weiteren sind im Gehäuse 51 drei Abstreifelemente 63, 65 angeordnet, deren Abstreifkanten 67 mit Reinigungsspitzen 69 des Reinigungsbesatzes 59 zusammenwirken. Hierdurch werden beispielsweise die Borsten oder Lamellen des Reinigungsbesatzes 59 lokal auseinandergespreizt, so dass der Schmutz 61 besser aus den dazwischenliegenden Lücken fallen kann und andererseits kann die elektromagnetische Strahlung tiefer zwischen die Borsten oder Lamellen vordringen. Des Weiteren bremsen die Abstreifelemente 63, 65 die Drehgeschwindigkeit des zylindrischen Grundkörpers 55, so dass eine Geschwindigkeitsdifferenz zwischen der Handlaufoberfläche 18 und den Reinigungsspitzen 69 entsteht. Dieser Sachverhalt ist in der Figur 4 symbolisch durch die ungleich langen Pfeile 71, 72 der Handlaufgeschwindigkeit des Handlaufs 17 und der Drehgeschwindigkeit des zylindrischen Grundkörpers 55 dargestellt.

Das Abstreifelement 65 kann eine Lippe aus biegeelastischem Material aufweisen, wobei an der Lippe die Abstreifkante 67 ausgebildet ist. Das Abstreifelement 63 kann aber auch eine Abstreifbürste sein, deren Borstenspitzen die Abstreifkante 67 bilden. Grundsätzlich können alle Abstreifelemente 63, 65 einer Handlauf-Behandlungsvorrichtung 41 dieselbe Ausgestaltung aufweisen. Wie in der Figur 4 klar ersichtlich ist, kann auch eine Kombination der beiden Ausführungsvarianten des Abstreifelements 63, 65 im selben Gerät eingesetzt werden.

Um die im Gehäuse 51 angesammelten Schmutzpartikel 61 entfernen zu können, kann eine Schmutzentfernungseinrichtung 75 vorhanden sein. Im vorliegenden Ausführungsbeispiel ist diese eine einfache Schublade, die durch das Servicepersonal von Zeit zu Zeit geleert werden kann. Selbstverständlich kann als Schmutzentfernungseinrichtung 75 am Gehäuse 55 auch ein Anschluss für eine Absaugvorrichtung vorhanden sein, um den anfallenden Schmutz 61 automatisiert kontinuierlich oder periodisch absaugen zu können.

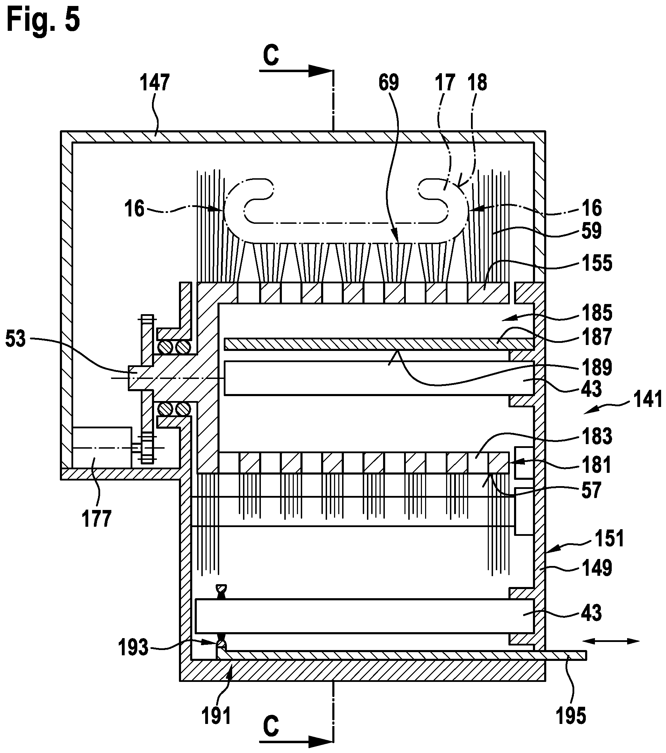

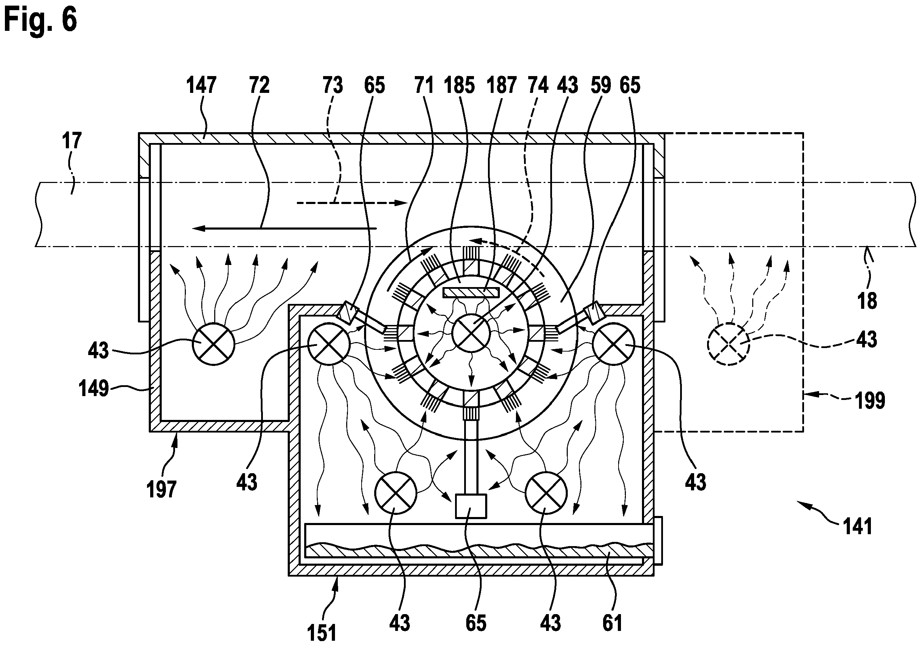

Die Figur 5 zeigt in einer Frontansicht die in den Figuren 1 und 2 dargestellte Handlauf-Behandlungsvorrichtung 141 als zweites Ausführungsbeispiel. Die Figur 6 zeigt ein Querschnitt entlang der Linie C-C durch die in der Figur 5 dargestellte Handlauf-Behandlungsvorrichtung 141. Nachfolgend werden die beiden Figuren 5 und 6 gemeinsam beschrieben.

Das zweite Ausführungsbeispiel weist wie das erste Ausführungsbeispiel ebenfalls mehrere gleichwirkende Komponenten wie ein Gehäuse 151 und ein darin um eine Drehachse 53 drehbar angeordneter, zylindrischer Grundkörper 155 auf. Auch Abstreifelemente 65 und Strahlungsquellen 43 für keimtötende, elektromagnetische Strahlung wie beispielsweise UV-C Licht sind im Gehäuse 151 angeordnet.

Einer der wesentlichen Unterschiede zum ersten Ausführungsbeispiel besteht darin, dass der zylindrische Grundkörper 155 durch einen Motor 177 angetrieben werden kann. Hierbei ist vorzugsweise die Drehgeschwindigkeit des zylindrischen Grundkörpers 155 derart auf die Geschwindigkeit des Handlaufs 17 abgestimmt, dass bei bewegtem Handlauf 17 zwischen der Handlaufoberfläche 18 und den diesen berührenden Reinigungsspitzen 69, eine Differenzgeschwindigkeit vorhanden ist. Gegebenenfalls kann auch eine der Handlaufoberfläche 18 entgegengesetzte Drehrichtung des Grundkörpers 155 gewählt werden, wie dies durch die mit ausgezogener Linie beziehungsweise unterbrochener Linie dargestellten Pfeile 71, 72, 73, 74 angedeutet ist.

Ein weiterer Unterschied besteht darin, dass der zylindrische Grundkörper 155 becherförmig ausgebildet ist und dessen Zylindermantel 181 Durchbrüche 183 aufweist. Der Reinigungsbesatz 59, im vorliegenden Ausführungsbeispiel Borsten, ist an der Zylinderaussenfläche 57 des vorhandenen Zylindermantels 181 angeordnet. Das Innere 185 des becherförmig ausgebildeten Grundkörpers 155 wird durch die darin angeordnete Strahlungsquelle 43 derart ausgeleuchtet, dass deren keimtötende elektromagnetische Strahlung durch die Durchbrüche 183 hindurch zum Reinigungsbesatz 59 gelangt.

Damit sich nicht Schmutz 61 schwerkraftbedingt auf der Oberseite der Strahlungsquelle 43 ansammelt, ist ein Schmutzschutz 187 für die Strahlungsquelle 43 im Gehäuse 151 angeordnet. Dieser kann zudem mit einer zur Strahlungsquelle 43 hin gerichteten, reflektierenden Fläche 189 versehen sein, so dass die auf die reflektierende Fläche 189 treffende Strahlung in andere Bereiche des Innern 185 des Grundkörpers 155 reflektiert wird.

Wie in der Figur 5 dargestellt, kann zudem für mindestens eine der Strahlungsquellen 43 eine Reinigungsvorrichtung 191 vorhanden sein. Diese ist beispielhaft als Bürstenring 193 ausgebildet, welcher mittels eines Betätigungsgestänges 195 über die Strahlungsquelle 43 bewegt werden kann. Vorzugsweise ist für jede der Strahlungsquellen 43 eine Reinigungsvorrichtung 191 und/oder ein Schmutzschutz 187 im Gehäuse 151 angeordnet.

Wie aus der Figur 6 ersichtlich ist, weist das zweite Ausführungsbeispiel der Handlauf-Behandlungsvorrichtung 141 eine dem zylindrischen Grundkörper 155 mit Reinigungsbesatz 59 nachgeschalteter Bereich 197, 199 mit Strahlungsquellen 43 auf. Dieser nachgeschaltete Bereich 197, 199 ist auf die Bewegungsrichtung des Handlaufs 17 abgestimmt, das heisst, er ist so angeordnet, dass die keimtötende elektromagnetische Strahlung von dessen Strahlungsquelle 43 den durch den Reinigungsbesatz 59 gereinigten Handlauf 17 bestrahlt. Sofern die Personentransportanlage 1 in beiden Förderrichtungen betrieben werden kann, können, wie durch die unterbrochenen Linien angedeutet, auch beidseits des Grundkörpers 155 solche nachgeschalteten Bereiche 197, 199 vorhanden sein. Beispielsweise können dann die Strahlungsquellen 43 je nach Förderrichtung der Personentransportanlage 1 angesteuert werden.

Um den Reinigungsbesatz 59 vor Umgebungseinflüssen zu schützen und, sofern vorhanden, die Strahlungsquellen 43 der nachgeschalteten Bereiche 197, 199 aus Sicherheitsgründen abzudecken, kann das Gehäuse 151 zweiteilig ausgeführt sein und ein Hauptgehäuseteil 149 mit einer offenen Seite und eine, die offene Seite des Hauptgehäuseteils 149 überspannende Abdeckung 147, aufweisen.

Bei beiden vorangehend beschriebenen Ausführungsbeispielen der Handlauf-Behandlungsvorrichtung 41, 141 ist zudem der Reinigungsbesatz 59 orthogonal zur Drehachse 53 profiliert, so dass eine Laufflächenkontur des Reinigungsbesatzes 49 mit einer Aussenflächenkontur des zu führenden Handlaufes 17 korrespondiert. Hierdurch können auch die Seitenflanken 16 des Handlaufs 17 gereinigt und desinfiziert werden.

Obwohl die Figuren 1 bis 6 unterschiedliche Aspekte der vorliegenden Erfindung anhand einer als Fahrtreppe ausgestalteten Personentransportanlage 1 zeigen, welche vertikal voneinander beabstandete Etagen E1, E2 miteinander verbinden soll, ist es offensichtlich, dass die beschriebene Handlauf-Behandlungsvorrichtung 41, 141 gleichermaßen auch bei schräg angeordneten Fahrsteigen oder horizontal angeordneten Fahrsteigen verwendet werden können. Zudem können die nachgeschalteten Bereiche 197, 199 jeweils auch mehrere Strahlungsquellen 43 aufweisen und in verschiedenen Ebenen angeordnet sein, so dass die gesamte, von Benutzern der Personentransportanlage berührbare Handlaufoberfläche 18 direkt von Strahlungsquellen bestrahlt werden können. Ferner ist an Stelle eines Motors 177 auch ein die Bewegung des Handlaufs 17 abgreifendes Reibrad einsetzbar, welches über ein unter- oder übersetzendes Getriebe den Grundkörper 155 antreiben kann.

Abschließend ist darauf hinzuweisen, dass Begriffe wie "aufweisend", "umfassend", etc. keine anderen Elemente oder Schritte ausschließen und Begriffe wie "eine" oder "ein" keine Vielzahl ausschließen. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Handlauf-Behandlungsvorrichtung (41, 141), umfassend zumindest ein Gehäuse (51, 151) und ein im Gehäuse (51, 151) um eine Drehachse (53) drehbar angeordneter, zylindrischer Grundkörper (55, 155), an dessen Zylinderaussenfläche (57) ein Reinigungsbesatz (59) angeordnet ist, über welchen Reinigungsbesatz (59) zwecks Entfernung von Schmutz und Keimen (61) ein umlaufender Handlauf (17) einer als Fahrtreppe oder Fahrsteig ausgestalteten Personentransportanlage (1) führbar ist, **dadurch gekennzeichnet, dass** innerhalb des Gehäuses (51, 151) mindestens eine Strahlungsquelle (43) angeordnet ist, die keimtötende elektromagnetische Strahlung emittieren kann, wobei die Strahlungsquelle (43) derart auf den Grundkörper (55, 155) ausgerichtet ist, dass bei drehendem Grundkörper (55, 155) der sich kontinuierlich an der Strahlungsquelle (43) vorbeibewegende Reinigungsbesatz (59) der keimtötenden elektromagnetischen Strahlung dieser Strahlungsquelle (43) ausgesetzt ist.

2. Handlauf-Behandlungsvorrichtung (41, 141) nach Anspruch 1, wobei im Gehäuse (51, 151) zumindest ein Abstreifelement (63, 65) vorhanden ist, dessen Abstreifkante (67) mit Reinigungsspitzen (69) des Reinigungsbesatzes (59) zusammenwirkt.

3. Handlauf-Behandlungsvorrichtung (41, 141) nach Anspruch 2, wobei das Abstreifelement (65) eine Lippe aus biegeelastischem Material aufweist und an der Lippe die Abstreifkante (67) ausgebildet ist.

4. Handlauf-Behandlungsvorrichtung (41, 141) nach Anspruch 2, wobei das Abstreifelement (63) eine Abstreifbürste aufweist, deren Borstenspitzen die Abstreifkante (67) bilden.

5. Handlauf-Behandlungsvorrichtung (41, 141) nach einem der Ansprüche 2 bis 4, wobei der zylindrische Grundkörper (55, 155) durch die Bewegung des über diesen geführten Handlaufs (17) angetrieben wird und das Abstreifelement (63, 65) derart mit den Reinigungsspitzen (69) des Reinigungsbesatzes (59) zusammenwirkt, dass bei bewegtem Handlauf (17) dadurch eine Bremswirkung am zylindrischen Grundkörper (55, 155) erfolgt und infolgedessen zwischen der Handlaufoberfläche 18 des Handlaufs (17) und den diesen berührenden Reinigungsspitzen (69) eine Differenzgeschwindigkeit vorhanden ist.

6. Handlauf-Behandlungsvorrichtung (41, 141) nach einem der Ansprüche 1 bis 4, wobei der zylindrische Grundkörper (55, 155) durch einen Motor (177) antreibbar ist und die Drehgeschwindigkeit des zylindrischen Grundkörpers (55, 155) derart auf die Geschwindigkeit des Handlaufs (17) abgestimmt ist, dass bei bewegtem Handlauf (17) zwischen der Handlaufoberfläche (18) des Handlaufs (17) und den diesen berührenden Reinigungsspitzen (69) eine Differenzgeschwindigkeit vorhanden ist.

7. Handlauf-Behandlungsvorrichtung (41, 141) nach einem der Ansprüche 1 bis 6, wobei der Reinigungsbesatz (59) orthogonal zur Drehachse (53) profiliert ist, so dass eine Laufflächenkontur des Reinigungsbesatzes (59) mit einer Aussenflächenkontur des zu führenden Handlaufes (17) korrespondiert.

8. Handlauf-Behandlungsvorrichtung (41, 141) nach einem der Ansprüche 1 bis 7, wobei der zylindrische Grundkörper (55, 155) becherförmig ausgebildet ist und dessen Zylindermantel (181) Durchbrüche (183) aufweist, wobei der Reinigungsbesatz (59) an der vorhandenen Zylinderaussenfläche (57) des Zylindermantels (181) angeordnet ist und wobei das Innere (185) des becherförmig ausgebildeten Grundkörpers (55, 155) durch die Strahlungsquelle (43) oder eine weitere Strahlungsquelle (43) derart ausleuchtbar ist, dass deren keimtötende elektromagnetische Strahlung durch die Durchbrüche (183) hindurch zum Reinigungsbesatz (59) gelangt.

9. Handlauf-Behandlungsvorrichtung (41, 141) nach einem der Ansprüche 1 bis 8, wobei ein Schmutzschutz (187) für die mindestens eine Strahlungsquelle (43) im Gehäuse (51, 151) angeordnet ist.

10. Handlauf-Behandlungsvorrichtung (41, 141) nach einem der Ansprüche 1 bis 9, wobei eine Reinigungsvorrichtung (191) für die mindestens eine Strahlungsquelle (43) im Gehäuse (51, 151) angeordnet ist.

11. Handlauf-Behandlungsvorrichtung (41, 141) nach einem der Ansprüche 1 bis 10, wobei bezogen auf eine Bewegungsrichtung des Handlaufs (17) eine dem zylindrischen Grundkörper (55, 155) mit Reinigungsbesatz (59) nachgeschalteter Bereich (197, 199) mit Strahlungsquellen (43) vorhanden ist, mittels deren keimtötenden elektromagnetischen Strahlung zumindest ein Teil der Handlaufoberfläche (18) des Handlaufs (17) bestrahlbar ist.

12. Handlauf-Behandlungsvorrichtung (41, 141) nach einem der Ansprüche 1 bis 11, wobei das Gehäuse (51, 151) zweiteilig ausgeführt ist und ein Hauptgehäuseteil (149) mit einer offenen Seite und eine, die offene Seite des Hauptgehäuseteils (149) überspannende Abdeckung (147), aufweist.

13. Personentransportanlage (1) die als Fahrtreppe oder Fahrsteig ausgebildet ist, mit mindestens einer Balustrade (15), die mindestens einen umlaufenden Handlauf (17) umfasst, **dadurch gekennzeichnet, dass** die Personentransportanlage (1) für jeden umlaufenden Handlauf (17) mindestens eine Handlauf-Behandlungsvorrichtung (41, 141) nach einem der Ansprüche 1 bis 12, aufweist.

14. Personentransportanlage (1) nach Anspruch 13, wobei die Balustrade (15) einen Balustradensockel (13) umfasst, in dem ein rückführendes Trum des Handlaufs (17) vor den Benutzern der Personentransportanlage (1) verdeckt, geführt ist und wobei die Handlauf-Behandlungsvorrichtung (41, 141) im Balustradensockel (13) der Balustrade (15) installiert ist.

15. Verfahren zum Steuern einer Handlauf-Behandlungsvorrichtung (41, 141) in einer Personentransportanlage (1) nach Anspruch 13 oder 14, **dadurch gekennzeichnet, dass** die Personentransportanlage (1) einen Antriebsmotor (21) zum Antreiben des Handlaufs (17) und eine Antriebssteuerung (19) zum Steuern des Antriebsmotors (21) aufweist, wobei die mindestens eine Strahlungsquelle (43) der Handlauf-Behandlungsvorrichtung (41, 141) abhängig von einem Geschwindigkeitssignal und/oder Bewegungsrichtungssignal der Antriebssteuerung (19) an den Antriebsmotor (21), angesteuert wird.

## Claims

1. Handrail treatment device (41, 141), comprising at least one housing (51, 151) and a cylindrical base body (55, 155) rotatably arranged in the housing (51, 151) about an axis of rotation (53), on the cylinder outer surface (57) of which a cleaning facing (59) is arranged, over which cleaning facing (59) a circulating handrail (17) of a passenger transport system (1) designed as an escalator or moving walkway can be guided for the purpose of removing dirt and germs (61), **characterized in that** at least one radiation source (43) is arranged inside the housing (51, 151), which radiation source can emit germicidal electromagnetic radiation, wherein the radiation source (43) is aligned with the base body (55, 155) in such a way that the cleaning facing (59) moving continuously past the radiation source (43) is exposed to the germicidal electromagnetic radiation of this radiation source (43) when the base body (55, 155) rotates.

2. Handrail treatment device (41, 141) according to claim 1, wherein there is at least one wiping element (63, 65) in the housing (51, 151), the wiping edge (67) of the wiping element interacting with cleaning tips (69) of the cleaning facing (59).

3. Handrail treatment device (41, 141) according to claim 2, wherein the wiping element (65) has a lip made of flexible material and the wiping edge (67) is formed on the lip.

4. Handrail treatment device (41, 141) according to claim 2, wherein the wiping element (63) has a wiping brush, the bristle tips of which form the wiping edge (67).

5. Handrail treatment device (41, 141) according to any of claims 2 to 4, wherein the cylindrical base body (55, 155) is driven by the movement of the handrail (17) being guided over it and the wiping element (63, 65) interacts in such a way with the cleaning tips (69) of the cleaning facing (59) that, when the handrail (17) is moved, a braking effect takes place on the cylindrical base body (55, 155) and, as a result, there is a differential speed between the handrail surface 18 of the handrail (17) and the cleaning tips (69) touching it.

6. Handrail treatment device (41, 141) according to any of claims 1 to 4, wherein the cylindrical base body (55, 155) can be driven by a motor (177) and the rotational speed of the cylindrical base body (55, 155) is adjusted to the speed of the handrail (17) so that there is a speed difference between the handrail surface (18) of the handrail (17) and the cleaning tips (69) touching it when the handrail (17) is moved.

7. Handrail treatment device (41, 141) according to any of claims 1 to 6, wherein the cleaning facing (59) is profiled orthogonally to the axis of rotation (53) so that a tread contour of the cleaning facing (59) corresponds to an outer surface contour of the handrail (17) to be guided.

8. Handrail treatment device (41, 141) according to any of claims 1 to 7, wherein the cylindrical base body (55, 155) is cup-shaped and the cylinder jacket (181) thereof has openings (183), wherein the cleaning facing (59) is arranged on the existing cylinder outer surface (57) of the cylinder jacket (181), and wherein the interior (185) of the cup-shaped base body (55, 155) can be illuminated by means of the radiation source (43) or a further radiation source (43) in such a way that its germicidal electromagnetic radiation is passed through the openings (183) to the cleaning facing (59).

9. Handrail treatment device (41, 141) according to any of claims 1 to 8, wherein a dirt protection (187) is arranged in the housing (51, 151) for the at least one radiation source (43).

10. Handrail treatment device (41, 141) according to any of claims 1 to 9, wherein a cleaning device (191) is arranged in the housing (51, 151) for the at least one radiation source (43).

11. Handrail treatment device (41, 141) according to any of claims 1 to 10, wherein, with reference to a direction of movement of the handrail (17), a region (197, 199) having radiation sources (43) downstream of the cylindrical base body (55, 155) having a cleaning facing (59) is present, at least part of the handrail surface (18) of the handrail (17) being able to be irradiated by means of germicidal electromagnetic radiation of the radiation sources.

12. Handrail treatment device (41, 141) according to any of claims 1 to 11, wherein the housing (51, 151) is designed in two parts and has a main housing part (149) having an open side and a cover (147) covering the open side of the main housing part (149).

13. Passenger transport system (1) which is designed as an escalator or a moving walkway, comprising at least one balustrade (15) which comprises at least one circulating handrail (17), **characterized in that** the passenger transport system (1) has at least one handrail treatment device (41, 141) for each circulating handrail (17) according to any of claims 1 to 12.

14. Passenger transport system (1) according to claim 13, wherein the balustrade (15) comprises a balustrade base (13) in which a return run of the handrail (17) is guided so as to be concealed from the users of the passenger transport system (1) and wherein the handrail treatment device (41, 141) is installed in the balustrade base (13) of the balustrade (15).

15. Method for controlling a handrail treatment device (41, 141) in a passenger transport system (1) according to either claim 13 or claim 14, **characterized in that** the passenger transport system (1) has a drive motor (21) for driving the handrail (17) and a drive controller (19) for controlling the drive motor (21), the at least one radiation source (43) of the handrail treatment device (41, 141) being controlled as a function of a speed signal and/or movement direction signal from the drive controller (19) to the drive motor (21).

## Revendications

1. Dispositif de traitement de main courante (41, 141), comprenant au moins un boîtier (51, 151) et un corps de base (55, 155) cylindrique disposé dans le boîtier (51, 151) de manière à pouvoir tourner autour d'un axe de rotation (53), une garniture de nettoyage (59) étant disposée au niveau de la surface externe cylindrique (57) dudit corps, une main courante (17) périphérique d'une installation de transport de personnes (1) conçue sous forme d'escalier roulant ou de trottoir roulant pouvant être guidée sur ladite garniture de nettoyage (59) en vue de l'élimination de souillures et de germes (61), **caractérisé en ce qu'**au moins une source de rayonnement (43) est disposée à l'intérieur du boîtier (51, 151), laquelle source peut émettre un rayonnement électromagnétique germicide, la source de rayonnement (43) étant orientée sur le corps de base (55, 155) de telle sorte que lorsque le corps de base (55, 155) est en rotation, la garniture de nettoyage (59) se déplaçant en continu devant la source de rayonnement (43) est exposée au rayonnement électromagnétique germicide de cette source de rayonnement (43).

2. Dispositif de traitement de main courante (41, 141) selon la revendication 1, au moins un élément de raclage (63, 65), dont le bord de raclage (67) interagit avec des pointes de nettoyage (69) de la garniture de nettoyage (59), étant présent dans le boîtier (51, 151).

3. Dispositif de traitement de main courante (41, 141) selon la revendication 2, l'élément de raclage (65) présentant une lèvre en matériau souple et le bord de raclage (67) étant réalisé au niveau de la lèvre.

4. Dispositif de traitement de main courante (41, 141) selon la revendication 2, l'élément de raclage (63) présentant une brosse de raclage, dont les pointes de poil forment le bord de raclage (67).

5. Dispositif de traitement de main courante (41, 141) selon l'une des revendications 2 à 4, le corps de base (55, 155) cylindrique étant entraîné par le déplacement de la main courante (17) guidée sur celui-ci et l'élément de raclage (63, 65) interagissant avec les pointes de nettoyage (69) de la garniture de nettoyage (59) de telle sorte que lorsque la main courante (17) se déplace, un effet de freinage se produit au niveau du corps de base (55, 155) cylindrique, suite à quoi une vitesse différentielle existe entre la surface 18 de la main courante (17) et les pointes de nettoyage (69) en contact avec celle-ci.

6. Dispositif de traitement de main courante (41, 141) selon l'une des revendications 1 à 4, le corps de base (55, 155) cylindrique pouvant être entraîné par un moteur (177) et la vitesse de rotation du corps de base (55, 155) cylindrique étant adaptée à la vitesse de la main courante (17) de telle sorte que lorsque la main courante (17) est en déplacement, une vitesse différentielle existe entre la surface (18) de la main courante (17) et les pointes de nettoyage (69) en contact avec celle-ci.

7. Dispositif de traitement de main courante (41, 141) selon l'une des revendications 1 à 6, la garniture de nettoyage (59) étant profilée orthogonalement par rapport à l'axe de rotation (53) de telle sorte qu'un contour de la surface de roulement de la garniture de nettoyage (59) correspond à un contour de surface externe de la main courante (17) à guider.

8. Dispositif de traitement de main courante (41, 141) selon l'une des revendications 1 à 7, le corps de base (55, 155) cylindrique étant réalisé en forme de gobelet et son enveloppe cylindrique (181) présentant des passages (183), la garniture de nettoyage (59) étant disposée au niveau de la surface externe cylindrique (57) existante de l'enveloppe cylindrique (181) et l'intérieur (185) du corps de base (55, 155) réalisé en forme de gobelet pouvant être éclairé par la source de rayonnement (43) ou une autre source de rayonnement (43) de telle sorte que son rayonnement électromagnétique germicide arrive, à travers les passages (183), jusqu'à la garniture de nettoyage (59).

9. Dispositif de traitement de main courante (41, 141) selon l'une des revendications 1 à 8, une protection contre les souillures (187) pour l'au moins une source de rayonnement (43) étant disposée dans le boîtier (51, 151).

10. Dispositif de traitement de main courante (41, 141) selon l'une des revendications 1 à 9, un dispositif de nettoyage (191) pour l'au moins une source de rayonnement (43) étant disposé dans le boîtier (51, 151).

11. Dispositif de traitement de main courante (41, 141) selon l'une des revendications 1 à 10, dans lequel, par rapport au sens de déplacement de la main courante (17), une zone (197, 199) comportant des sources de rayonnement (43) disposée en aval du corps de base (55, 155) cylindrique comportant la garniture de nettoyage (59) étant présente, au moins une partie de la surface (18) de la main courante (17) pouvant être irradiée à l'aide du rayonnement électromagnétique germicide desdites sources.

12. Dispositif de traitement de main courante (41, 141) selon l'une des revendications 1 à 11, le boîtier (51, 151) étant conçu en deux parties et présentant une partie principale de boîtier (149) comportant un côté ouvert et un recouvrement (147) couvrant le côté ouvert de la partie principale de boîtier (149).

13. Installation de transport de personnes (1) réalisée sous forme d'escalier roulant ou de trottoir roulant, comportant au moins une balustrade (15), qui comprend au moins une main courante (17) périphérique, **caractérisée en ce que** l'installation de transport de personnes (1) présente, pour chaque main courante (17) périphérique, au moins un dispositif de traitement de main courante (41, 141) selon l'une des revendications 1 à 12.

14. Installation de transport de personnes (1) selon la revendication 13, la balustrade (15) comprenant un socle de balustrade (13), dans lequel est guidé, de manière couverte pour les utilisateurs de l'installation de transport de personnes (1), un brin en retour de la main courante (17) et le dispositif de traitement de main courante (41, 141) étant installé dans le socle de balustrade (13) de la balustrade (15).

15. Procédé pour la commande d'un dispositif de traitement de main courante (41, 141) dans une installation de transport de personnes (1) selon la revendication 13 ou 14, **caractérisé en ce que** l'installation de transport de personnes (1) présente un moteur d'entraînement (21) destiné à entraîner la main courante (17) et une commande d'entraînement (19) destinée à commander le moteur d'entraînement (21), l'au moins une source de rayonnement (43) du dispositif de traitement de main courante (41, 141) étant commandée en fonction d'un signal de vitesse et/ou d'un signal de sens de déplacement de la commande d'entraînement (19) au moteur d'entraînement (21).
